# EUROPEAN PATENT APPLICATION

(11) **EP 4 357 296 A1**
(43) Date of publication of application: **24.04.2024**
(21) Application number: 22201973.9
(22) Date of filing: 17.10.2022
(51) Int. Cl.: B81C 1/00, A61J 3/00

(54) **PROCESS FOR THE MANUFACTURING OF MICROSCALE HOLLOW METAL BODIES**

(71) Applicant: Max-Planck-Institut für Eisenforschung GmbH, 40237 Düsseldorf (DE)
(72) Inventor: Ramachandramoorthy, Rajaprakash, 40472 Düsseldorf (DE); Kang, Sung-Gyu, 40233 Düsseldorf (DE)
(74) Representative: Janke Scholl Patentanwälte PartG mbB

(57) **Abstract**

A hollow body and a process for preparing a hollow body having a bottom, an upper end wall and side walls extending between the bottom and the upper end wall, characterized in that the upper end wall and side walls are made of metal, the cavity within the hollow body has a volume in the range between 1 picoliter to 1,000 picoliter and the upper end wall comprises at least one opening. The hollow body are suitable for encapsulating small amounts of liquid. or of very small micro-electromechanical system, may be used for the controlled release of pharmaceutical components.

## Description

The present invention relates to a hollow body made of metals obtained by an additive manufacturing process, a process for the manufacturing of the hollow body by an additive manufacturing process and the use of the hollow body for the storage of liquids in the cavity of the hollow body and the release of liquids from the cavity of the hollow body.

Microfabrication using classic UV lithography is suited only for creating two-dimensional structures with topography (2.5D) on very specific substrates such as silicon, which is intrinsically brittle, and a few thin films such as nickel and aluminium nitride. They also typically lack built-in damage sensing or impact protection mechanisms. As such, there is a critical need to identify micromanufacturing techniques that are capable of fabricating true 3D microarchitectures (size scale - smallest dimension: 200nm - 15µm). Recently, the advent of two-photon lithography (TPL) has enabled the 3D printing of microscale architectures. TPL uses a pulsed femtosecond laser beam with infra-red (IR) wavelength to define freeform3D polymer structures. Using TPL, a variety of complex microlattice designs and structures with nanometer resolution have been realized previously, but given that they are still polymer structures, they were inherently weak.

Liquid encapsulation in microscale architecture has a myriad of applications including drug delivery, impact protection, damage sensing etc. But even after 3-4 decades of silicon based microfabrication, liquid encapsulation remains largely out of reach. Only one previous academic exercise has partially achieved such encapsulation but requires extremely complex experimental steps.

Localized electrodeposition in liquid (LEL) is an additive micromanufacturing technique that uses a hollow atomic force microscope tip with a small orifice immersed in a standard three-cell electrochemical cell. Electrochemical ink with metal ions is fed through the tip and they get reduced locally on the working electrode, i.e. the conductive substrate. The localized electrodeposition based additive manufacturing technologies have the potential to fabricate 3D metal microarchitectures. Yet, owing to the complexity of the manufacturing process, excepting simple microneedles, complex architectures such as microlattices, micropillars, microsprings, etc., have not been realized using these techniques.

Object of present invention is to provide a process for preparing a hollow body made of metal having a cavity in which liquids can be encapsulated by localized electrodeposition and a hollow body obtained by such process.

Subject of present invention is a hollow body having a bottom, an upper end wall and side walls extending between the bottom and the upper end wall, characterized in that
- the upper end wall and the side walls are made of metal,
- the cavity within the hollow body has a volume in the range between 1 picoliter to 1,000 picoliter and
- the upper end wall comprises at least one opening.

A further object is a process for the preparation of the hollow body by localized electrodeposition in which a metal is deposited from an electrolyte solution containing metal ions on a substrate.

The hollow body according to present invention is manufactured by an additive manufacturing process, preferably by localized electrodeposition in liquid (LEL). The LEL technique has the advantage that multiphase printing is possible - as liquids can be potentially encapsulated within metal cavities and the voxel diameter can be controlled scaled by using pressure changes (without change of tip aperture). LEL is a voxel by voxel deposition method allows freeform printing of complex microscale geometries with submicron resolution. It is possible to obtain microarchitecture such as hollow bodies that are structural error free, have low surface roughness, and are fully dense. It is possible to manufacture hollow bodies of any external shape (simple cylinders to complex 3D architectures such as lattices). The shape may be selected depending on the requirements of the area of application. In most applications the hollow body has a simple geometric form such as a cubic, rectangular-shaped or cylindrical form. Usually the shape of the hollow body according to present invention depends on the intended application release of liquids in general, a simple cylindrical shape would suffice. But for example, if multifunctionality of impact protection and damage sensing is required, then in that case the hollow shape would be much more complex such as a microlattice geometry, i.e. an architected design with typically repeating unit cells of the same design (compared to randomly shaped foams for example.

The microlattice architecture provide the impact protection, while the encapsulated liquid (with a fluorescence marker) would provide both added impact protection and damage sensing mechanism, using quick fluorescence microscopy inspection.

According to the process of present invention it is possible to prepare micro architectures with dimensions up to 1 mm. The walls may have a thickness from 1 µm to 15 µm, preferably from 2 µm to 10 µm. The cavity inside the hollow body may have a volume from 1 picoliter to 1,000 picoliter, preferably from 10 femtoliter to 100 picoliter. In this cavity liquids and/or MEMS (micro-electromechanical system) may be encapsulated. The liquids may be selected from any kind of pharmaceuticals and pharmaceutical containing liquids.

The hollow body according to present invention is made of metal. Any metal that can be electrodeposited from an aqueous solution can be used to manufacture metal microarchitectures using the LEL process, such as Cu, Ag, Au, Pd, Pt, Co, Ni, Fe and also rare earth metals such as In.

In a preferred embodiment of present invention the bottom of the hollow body has the form of a flat body or disc and is made from a conductive material or coated with a conductive material so that it can be used as the working electrode (or counter electrode) in a LEL process. Examples of conductive materials are metals, conductive polymer material including carbon-fibre-reinforced polymers, a wafer made of glass or any other material coated with conductive materials. In one embodiment of present invention the bottom of the hollow body is prepared during the electrodeposition process, i.e. the layers of the bottom are prepared first. In another embodiment, the substrate used in the electrodeposition process will be the bottom of the finished product. Depending on the intended use of the finished hollow body, the substrate may be removed after the end of the process.

In a further embodiment of present invention the substrate can be a non-conductive substrate, i. e. an insulative substrate, which comprises conductive prints. The conductive wires are drawn from a surrounding conductive working electrode, for example a graphite electrode. The insulative substrate may be arranged on a conductive substrate which is connected to the working electrode.

The upper end wall covers the cavity of the hollow body and has at least one opening over which encapsulated liquid can be released. In case the hollow body comprise more than one opening in the upper end wall, these openings can be distributed over the area of the upper end wall. The upper end wall is usually made of the same material as the side walls extending between the bottom and the upper end wall. The opening can correspond to the opening area of the cavity which may be covered with another material after filling liquid or any other components into the cavity.

In one embodiment of present invention the upper end wall is covered with a thin film, preferably with a polymer, for example a biodegradable polymer.

The printing variables that require optimization based on the electrodeposition and geometry choice include deposition voltage, air pressure and the orifice diameter. The localized electrochemical deposition process requires a supporting electrolyte solution for achieving the conductive path between the working and counter/reference electrode, which forms the three cell electrochemical setup. Using the voxel by voxel deposition submerged inside this supporting electrolyte liquid (aqueous solution of pH 2 with sulphuric and hydrochloric acid), closed form geometries such as cylinders or shells when printed will have the supporting conductive electrolyte (SCE) sealed within the void. Specifically for sealing liquids successfully within microscale metallic shells, it's also vital to optimize the voxel overlap between adjacent voxels in the layer of printing and within the layers themselves. This prevents the leakage of the encapsulated liquids through small pores.

During the fabrication process, the overpotential can be varied between -0.49V to -0.51V.

The ionic solution can be fed to the container with a certain pressure which can be varied between 20mbar to 60mbar.

Voxel locations were determined from the computer-aided design based model of metal microarchitectures. A distance between each voxel can be chosen from 0.3 µm to 2.0 µm, preferably from 0.5 µm to 1.5 µm, to prevent micro/nano voids between deposited metal voxels.

Currently, LEL remains one of the two techniques that have the potential to encapsulate liquids, as the fabrication process needs to be conducted submerged in liquid. The other technique which theoretically has the potential to encapsulate liquids similarly is laser induced photoreduction. But is plagued with issues of very high surface roughness and porosity making it currently not suitable again for encapsulation of liquids within metal micro scale architectures, such as hollow bodies which are microvessels for the liquids.

The electrochemical deposition process can be customized to a certain extent (within the electrodeposition voltage window with no hydrogen evolution process) by changing the pH and composition of the supporting electrolyte solution, especially for the deposition of different metals. For example, a copper deposition requires typically a pH of between 2-3 in the SCE, while gold deposition requires an alkaline pH of 12. And the low concentration of nanoparticulates (such as nanoparticles, nanosheets) or biological moieties (that won't degrade in the pH conditions in the SCE) which won't influence the deposition process, can be introduced in the SCE and can in turn be sealed within the metal microarchitectures.

As mentioned before the electrolyte solution is selected depending on the metal to be deposited. In case the metal is deposited at a pH below 7, i. e. in an acidic solution, for example a mixture of HCI and H₂SO₄, is used. In case the metal is deposited at a pH above 7, preferably above 10, i.e. in an alkaline solution is used, for example an aqueous solution of NaOH and optional KCI and/or Na₂SO₄

To circumvent the restriction of sealing only the SCE in the metal microarchitectures, hollow architectures with a microscale opening could be fabricated. Later after the manufacture of such architectures, other liquids such as liquids with different viscosities or drugs could be dispensed using the same hollow AFM tip, to fill the voids. Further, these holes on the microarchitectures could be sealed via local deposition or spin coating of polymers such as PLGA. This will enable the possibility to even have timed release of drugs in human biological conditions, as PLGA will degrade over time and encapsulated drugs will be released.

Another subject of present invention is use of the hollow body for the controlled release of pharmaceutical components.

In the enclosed figures present invention is explained in more detail. The figures show in
Figs. 1 and 1A a microvessel according to present invention with a hole in the upper wall end
Fig. 2 a 2D plot (x-y plot) of a view on top of the microvessel
Fig. 3 a 3D plot of the printed microvessel
Fig. 4 a schematic view of the LEL process
Fig. 5 Filling of liquid into the cavity of the microvessel
Fig. 6 Local sealing of the microvessel
Fig. 7 cross-section of a microvessel filled with liquid and coated with a polymer
Fig. 8 cross-section of the microvessel of Fig. 7 showing the release of the liquid after degradation of the polymer;
Fig. 9 microvessel filled with liquid and connected with a polymer substrate;
Fig. 10 cross-section of a microvessel with an encapsulated MEMS;
Fig. 11 Arrangement of the substrate and working electrode using an insulative substrate.

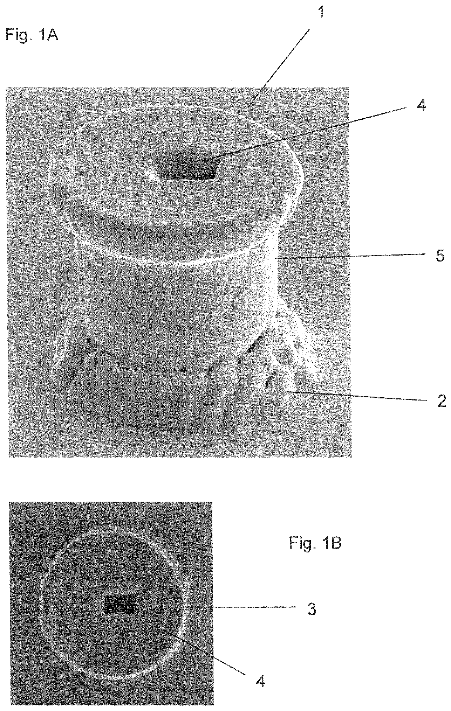

The microvessel shown in Fig. 1 is an example of an hollow body 1 according to present invention. The microvessel has cylindric form, the bottom 2 and the upper end wall 3 are thin discs. The upper end wall 3 comprises a hole 4 through which the liquid can be filled in and through which the liquid can be released into the cavity 5.

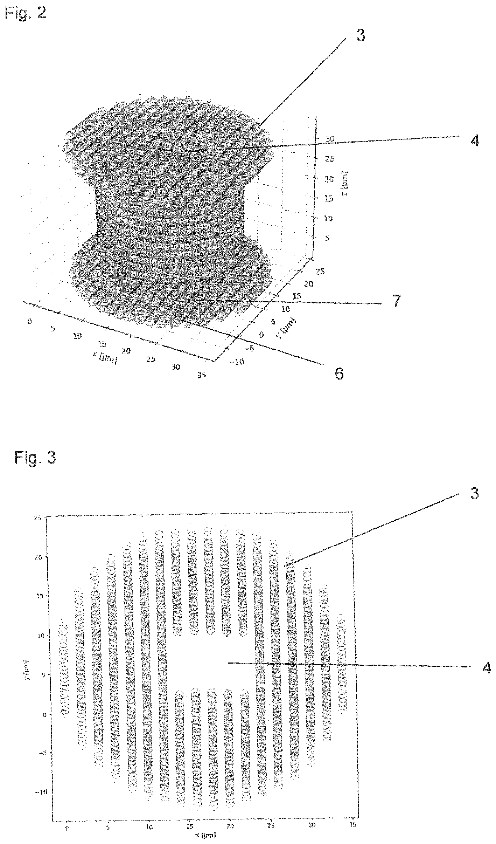

The preferred method for preparing the hollow body 1 of present invention is an additive micromanufacturing technique, especially the localized electrodeposition in liquid. A schematic view on top of the microvessel a x-y plot is shown in Fig. 2 and in Fig. 3 as a 3D plot. As it can be seen from the figures, the vessel comprises a bottom 2consisting of 2 layers 6, 7 ). First the lower bottom layer 6is printed and afterwards the second layer 7followed by the side walls 8. Finally the cavity (not shown in this figure) is closed by an upper cover 3. In the embodiment shown in Fig. 2 and 3 the bottom and the upper end walls are arranged parallel to each other.

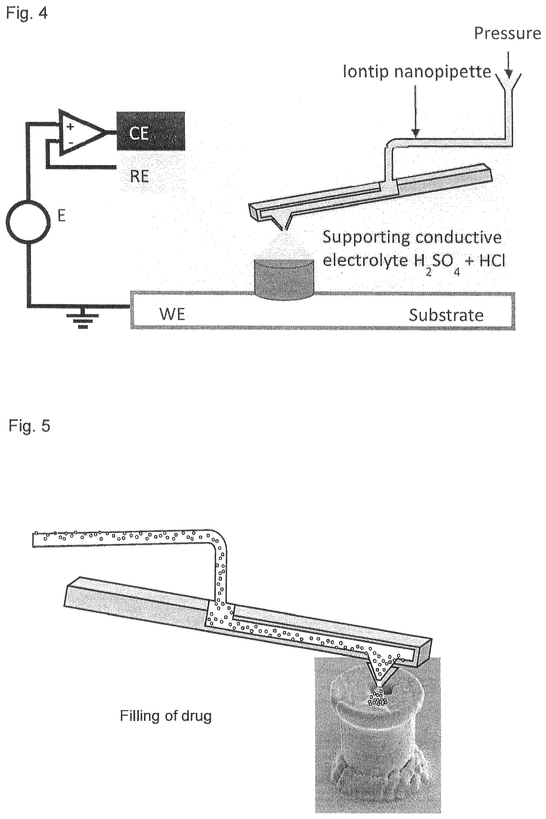

Fig. 4 shows schematic of the LEL process proceeding. The arrangement consists of an electrolytic cell with a reference electrode, a counter electrode and a working electrode. The voxel grow on the working electrode which is the substrate for the to be printed body. The ionic ink is the starting material for manufacturing the body and is supplied via the reservoir and a nanotip nanopipette to the substrate. The amount of the ionic ink and the speed of supply of the ink can be controlled by suitable electronic devices (not shown). Further, the geometry of the body and thickness of the walls can be achieved by adjusting several process parameters such as deposition voltage, air pressure and the orifice diameter.

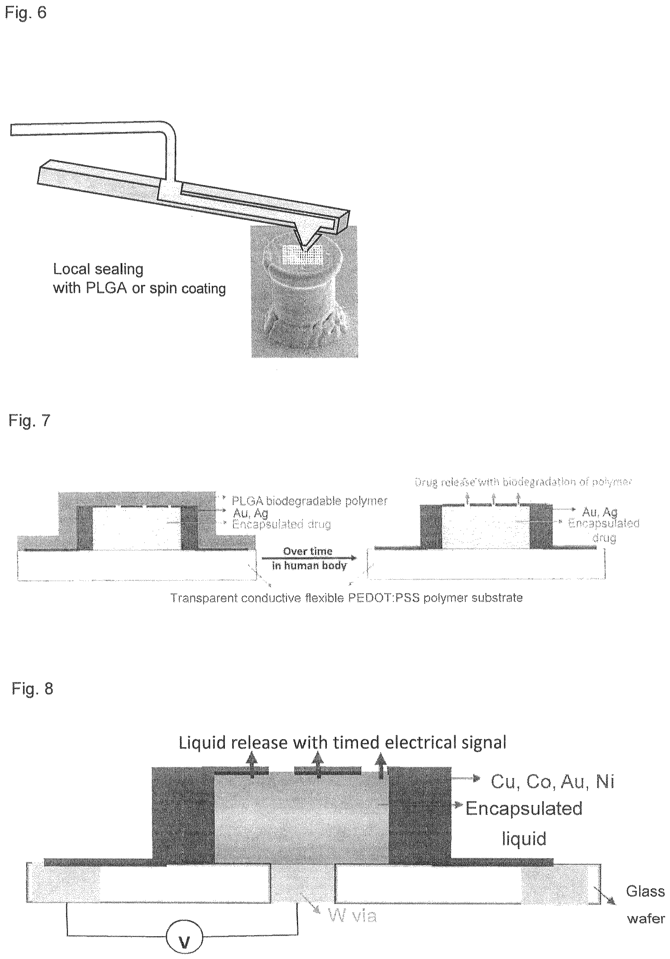

In a next step the obtained microvessel can be filled with a liquid, for example a drug (s. Fig.5), afterwards the hole(s) in the upper end wall are closed, for example by local sealing with a polymer, coating area of the hole(s) and around the hole(s) with a polymer, or coating more than only this area by coating the area of the upper end wall whereby the coating may extend also to the side walls (Fig. 6). Preferably, a biodegradable polymer is used for closing or covering the holes, for example polymers on the base of poly lactic acid and poly glycolic acid.

Fig. 7 shows a schematic view of a liquid, for example a pharmaceutical active agent, encapsulated in a hollow body according to present invention. The side walls and upper end walls are grown voxel by voxel on the working electrode which is bottom in the final product. The liquid is stored in the cavity of the hollow body. In order to prevent the liquid from leaving the cavity the holes in the upper end wall a closed, i. e. in the embodiment shown in Fig. 7 the upper end wall, the side walls and also part of the bottom and of the substrate are covered with a coating of an biodegradable polymer. When administered as a pharmaceutical agent to a human body, the biodegradable polymer will disintegrate and the closed holes are opened so that the pharmaceutical active is released.

Fig. 8 shows another embodiment of present invention. The bottom of the microvessel is a glass wafer with isolated electrical contacts (commercially available as conductive vias on glass substrates) which act as the working electrode in the production process. The release of the encapsulated liquid is initiated by an electrical signal. The voltage source is connected with the encapsulate liquid and the side walls and upper end walls and in case the circuit is closed the liquid will be released.

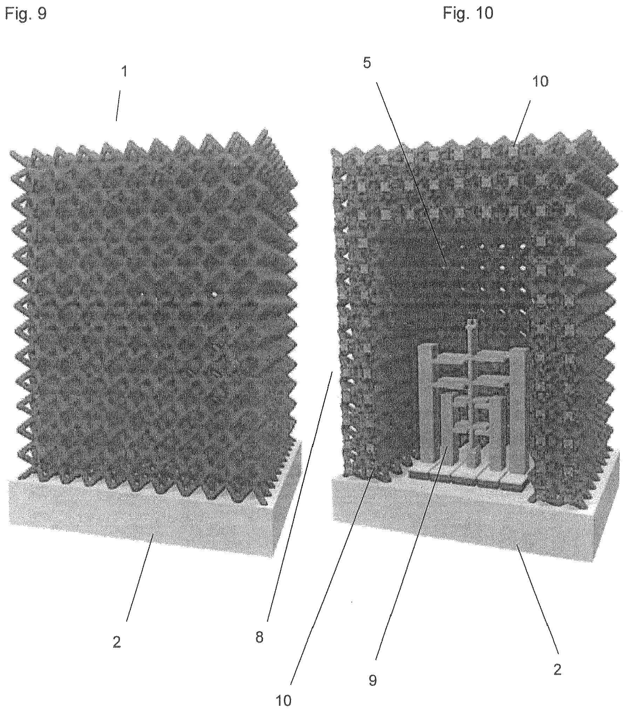

In Figs 9 and 10 the hollow body of present invention is shown in the form of the lattices of the deposited metal. Fig. 9 shows an external view of the hollow body according to present invention. Fig. 10 is a sectional view of the hollow body wherein a micro-electromechanical system 9 is arranged in the cavity 5 of the hollow body. In this embodiment the side walls 8 and the upper cover with walls 3 of lattice architecture comprise a luminescence marker. The marker is located in the lattice of the deposited metal and via this marker any damage to the metal lattice (for example: external particle impacts) can be identified using fluorescence microscopy. In this figure the lattices surrounding the MEMS indeed have porous (as lattices do have low density - which also provide impact protection) unit cells but the struts in the unit cells are themselves hollow encapsulating the liquid with fluoresence dye (required for both enhancing impact protection capability and providing fluoresence based damage sensing when there is an impact/crack etc). In essence, lattices are porous but are themselves hollow with the encapsulating liquid and the lattice structures are the packaging that protects the MEMS in the middle.

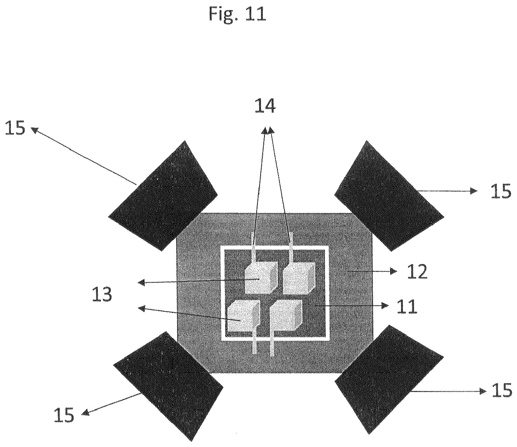

Fig. 11 shows an arrangement for the production of the hollow body using an insulative substrate 11. External graphite electrodes 15 are used as working electrodes. The insulative substrate 11 is applied on a conductive substrate 12. Electrically isolated 3D prints 13 are arranged on the insulative substrate 11 and connected with the working electrode 15 via conductive wires 14.

### Example

Liquid containing metal microarchitectures were fabricated via localized electrodeposition in liquid using the CERES system (Exaddon AG, Switzerland). A hollow atomic force microscope tip with a 300-nm-diameter orifice was used. The supporting electrolyte was a solution of 0.5M H₂SO₄ and 0.5 mM HCI. The plating solution was a 0.8M CuSO₄ in 0.5M H₂SO₄ solution." The process was carried out at room temperature, the applied overpotential was -0.5 V, and the pressure 60mbar. Voxel locations were determined from the computer-aided design based model of metal microarchitectures. A distance between each voxel was chosen to be 0.5 µm.

As a substrate a 15 mm x 15 mm Si-Ti-Cu substrate, which is basically silicon wafer with Ti layer (13 nm) and Cu layer on top (100 nm).

### List of reference numbers

- 1: hollow body
- 2: bottom
- 3: upper cover wall
- 4: hole
- 5: cavity
- 6: lower layer of the bottom 2
- 7: second layer of the bottom 2
- 8: side wall
- 9: MEMS
- 10: liquid with fluorescence marker
- 11: insulative substrate
- 12: conductive substrate
- 13: electrically isolated 3D prints
- 14: conductive wires
- 15: working electrodes

## Claims

1. Hollow body having a bottom, an upper end wall and side walls extending between the bottom and the upper end wall,
**characterized in that**
- the upper end wall and side walls are made of metal,
- the cavity within the hollow body has a volume in the range between 1 picoliter to 1,000 picoliter and
- the upper end wall comprises at least one opening.

2. Hollow body according to claim 1, **characterized in that** the metal is selected from Cu, Ag, Au, Pd, Pt, Co, Ni, Fe, and rare earth metals.

3. Hollow body according to claim 1, **characterized in that** the bottom is made of metal, a conductive polymer material including carbon-fibre-reinforced polymers, a wafer made of glass, or any other material coated with conductive layer.

4. Hollow body according to any of claims 1 to 3 **characterized in that** at least the upper end wall is covered with a polymer coating.

5. Hollow body according to any of claims 1 to 4 **characterized in that** the hollow body has a cubic, rectangular-shaped or cylindrical form.

6. Hollow body according to any of claims 1 to 5 **characterized in that** the bottom and the upper end walls are arranged parallel to each other.

7. Hollow body according to any of claims 1 to 6 **characterized in that** at least part of the walls comprise a luminescence marker.

8. Hollow body according to any of claims 1 to 7 **characterized in that** it is manufactured by an additive manufacturing process.

9. Process for the manufacturing a hollow body having at least side walls and an upper end wall made of metal and having a nanoscale size, which has a cavity within the hollow body has a volume in the range between 1 picoliter to 1,000 picoliter and wherein the additive upper cover comprises at least one opening by localized electrodeposition on a substrate which a metal is deposited from an electrolyte solution containing metal ions on a substrate.

10. Process according to claim 9 **characterized in that** liquids and/or micro-electromechanical system is encapsulated.

11. Process according to claim 10 **characterized in that** the liquid contains pharmaceutical components.

12. Use of the hollow body according to any of claims 1 to 8 for the controlled release of pharmaceutical components.
